# EUROPEAN PATENT APPLICATION

(11) **EP 0 922 760 A2**
(43) Date of publication of application: **16.06.1999**
(21) Application number: 98122542.8
(22) Date of filing: 01.12.1998
(51) Int. Cl.: C12N 15/00, A01K 67/027, A61K 49/00

(54) **Transgenic animal not expressing endogenous osteoclastogenesis inhibitory factor (OCIF)**

(30) Priority: 02.12.1997 JP 33224097
(71) Applicant: SNOW BRAND MILK PRODUCTS, CO., LTD., Sapporo-shi Hokkaido 065-0043 (JP)
(72) Inventor: Mizuno, Atsuko, Kiryu-shi, Gunma (JP); Murakami, Akihiko, Shimotsuga-gun, Tochigi (JP); Fujise, Nobuaki, Ishibashi-machi, Shimotsuga-gun, Tochigi (JP); Sato, Yasushi, Utsunomiya-shi, Tochigi (JP); Kanno, Takeshi, Utsunomiya-shi, Tochigi (JP); Tsuda, Eisuke, Daia-paresu, Minamikawachi-machi, Kawachi-gun Tochigi (JP); Morinaga, Tomonori, Shimatsuga-gun, Tochigi (JP); Higashio, Kanji, Kawagoe-shi, Saitama (JP)
(74) Representative: Forstmeyer, Dietmar, Dr. rer. nat., Dipl.-Chem.

(57) **Abstract**

The present invention relates to a novel transgenic mouse. It relates to providing transgenic animals which can not express endogenous osteoclastogenesis inhibitory factor, more specifically transgenic animals which will suffer from spontaneous metabolic bone disease, more specifically spontaneous osteoporosis. Further, a method of screening agents for preventing and/or treating metabolic bone diseases characterized by using transgenic animals of the present invention.

The transgenic animals of the present invention are useful for research of bone metabolism or for animals for research of evaluation and screening of agents for preventing and/or treating metabolic bone diseases.

## Description

### Technical field of the Invention

The present invention relates to transgenic animals which can not express endogenous osteoclastogenesis inhibitory factor, more specifically, transgenic animals which suffer from metabolic bone diseases, more specifically, osteoporosis. Further, the present invention relates to a method of screening for agents for preventing and/or treating metabolic bone disease, characterized by using the transgenic animal of the present invention.

The transgenic animals of the present invention are ideal model animals which are produced by the mechanism involving osteoclastogenesis and show a phenotype quite similar to human osteoporosis. Further, because the mortality rate due to bone fracture of limb immediately after birth is very low and the survival rate thereof is high, the model animals can grow normally to obtain fertility and can be bred.

Therefore, passage of animal models with spontaneous osteoporosis can be carried out easily by breeding once transgenic animals are made. Since these animals have advantages as described above, they are useful for research on bone metabolism and/or as test animals for evaluating and/or screening agents preventing and/or treating metabolic bone diseases.

### Background technique

Bone metabolism depends on total activities of osteoblasts in charge of bone formation and osteoclasts in charge of bone resorption. Anomaly of bone metabolism is thought to develop due to imbalance between bone formation and bone resorption. As a disease accompanying with anomaly of bone metabolism, osteoporosis, hypercalcemia, bone Paget disease, renal osteodystrophy, rheumatoid arthritis or osteoarthritis can be exemplified. As atypical metabolic bone disease, osteoporosis can be exemplified. This disease will develop due to exceeding of bone resorption by osteoclasts over bone formation by osteoblasts and have a feature of significant decrease in bone mineralization and bone matrix.

The mechanism to develop the disease has not been elucidated completely yet. The diseases causes bone pain and bone fracture due to fragile bone. With the increase in the population of the aged people, this disease causing bedridden old person due to bone fracture has become one of serious social problems and, hence, development of therapeutic agents for the disease is an urgent necessity. Osteopenia caused by anomaly of bone metabolism can be expected to be treated by suppressing bone resorption, stimulating bone formation or improving the balance between them. That is, bone formation can be expected to be enhanced by stimulating proliferation, differentiation and function of osteoblasts in charge of bone formation, suppressing differentiation and maturation of precursor cells of osteoclasts to osteoclasts or suppressing bone resorptive activities of osteoclasts.

At present, investigative and developmental researches on hormones, low molecular weight substances or biologically active proteins as given above are being carried out vigorously. As agents for treatment of bone related diseases or for shortening treatment duration thereof, calcitonin, active vitamin D₃, hormone comprising estradiol, ipriflavone, vitamin K₂, bisphosphonates are already used clinically and clinical tests on active vitamin D₃ derivatives, estradiol derivatives and the second generation or the third generation bisphosphonate compounds are being carried out for development of a therapeutic agent with lower side effects and higher efficacy.

However, as treatment with these pharmaceutical agents can not be necessarily satisfiable with respect to their efficacy and treatment results, development of a novel therapeutic agent with higher safety and efficacy are desirable.

In addition, application of some agents among those used in the treatment of metabolic bone diseases is restricted to specific diseases due to side effects thereof. Further, multiple drug combination therapy using multiple drugs at the same time become the mainstream of the treatment of metabolic bone diseases such as osteoporosis. From these points of view, development of a medicine having a novel action mechanism, higher efficacy and lower side effects than conventional medicines is expected.

As described above, cells in charge of bone metabolism are osteoblasts and osteoclasts. These cells are known to interact with each other closely. The functional cooperation between osteoblasts and osteoclasts is called "coupling". It was reported that cytokines, interleukin-1 (IL-1), -6 (IL-6), -11 (IL-11), granulocytic macrophage stimulating factor (GM-CSF), macrophage colony colony stimulating factor (M-CSF), interferon gamma, (IFN-α), TNF-α, transforming growth factor β (TGF-β) etc. secreted from osteoblastsic stromal cells stimulate or suppress differentiation and maturation of osteoclasts (Raisz: Disorders of Bone and Mineral Metabolism, 278-311, 1992; Suda et al.: Principles of Bone Biology, 87-102, 1996; Suda et al.: Endocrine Reviews, 4, 226-270, 1995; Lacey et al.: Endocrinology, 136, 2367-2376, 1995). Osteoblastic stromal cells are known to play an important roll in differentiation and maturation of osteoclasts and in activation of mature osteoclasts to resorb bone, by adhering to osteoclasts precursor cells and osteoclast, respectively. As a factor involved in osteoclastogenesis througth this cell-to-cell interaction, a molecule called osteoclasts differentiation factor (ODF), expressed on the membrane of osteoblastsic stromal cells is proposed and, based on this proposition, receptor for ODF is supposed to be present in osteoclasts precursor cell (Suda et al.: Endocrine Rev. 13, 68-80, 1992; Suda et al.: Bone 17, 87S-91S, 1995).

However, neither ODF nor the receptor thereof has been purified or identified. There was no report on their properties, action mechanism or structure. Thus, mechanism of differentiation and maturation of osteoclasts was not fully elucidated yet. Therefore, elucidation of the mechanism is expected to contribute significantly not only to the progress of basic medical science but also to development of a novel therapeutic agent for metabolic bone diseases, based on a novel action mechanism.

Under these circumstances, the present inventors have eagerly investigated and already found osteoclastogenesis inhibitory factor (OCIF) in the culture medium of human embryonic lung fibroblast IMR-90 (ATCC CCL186) WO96/26217). The present inventors continued investigation and succeeded in cloning of cDNA of OCIF, production of recombinant OCIF (rOCIF) and confirmation of pharmacological effects of rOCIF in vivo (improving effects on bone metabolism) (WO96/26217). When about 10 times and 100 times the pharmacological effect-expressing dose (effective dose) of rOCIF were administered intravenously in normal rats once a day for consecutive 14 days, bone density and bone volume were significantly augmented to raise osteopetrosis-like symptoms without any change in clinical chemical parameters in blood and urine, hematological parameters and histology in various organs except bone tissue. From the results, OCIF was found to act only on bone tissue and stimulate bone formation by suppressing specifically differentiation and maturation of osteoclasts.

Recently, cell-biological and molecular-biological research on the mechanism of bone metabolism have been progressed remarkably and pathological elucidation of osteoporosis is expected. The present inventors succeeded in cloning cDNA of target molecule (OCIF binding molecule, OBM) from a cDNA library of osteoblastsic stromal cell line ST2 by the method of expression cloning using OCIF, in order to elucidate the action mechanism of OCIF. It was elucidated that OBM encoded by OBM cDNA was a type II membrane bound protein and a factor supporting and stimulating the differentiation and maturation of osteoclasts in osteoclastogenesis in vitro (mouse OBM, Japanese patent application No. 217897/1997; human OBM, Japanese patent application No. 224803/1997).

The relationship between OCIF and OBM in osteoclastogenesis will be described briefly below. As an in vitro culture system to examine osteoclastogenesis, a co-culture of mouse osteoblastic stromal cell line ST2 and mouse spleen cells in the presence of active vitamin D₃ and dexamethasone can be exemplified. Using this culture system, the following things were confirmed: the presence of osteoblastic stromal cell line ST2 cells was essential; osteoclasts were formed in a culture of mouse spleen cells on immobilized monkey kidney cell line COS-7 expressing cDNA of OBM, in the presence of conditioned medium of ST2 cells or M-CS; addition of OCIF suppressed this osteoclastogenesis completely; in the presence of OCIF or, in the case of COS-7 cells with a vector which did not comprise cDNA of OBM, osteoclastogenesis was not observed.

The present inventors expressed sOBM cDNA encoding soluble OBM (sOBM) lacking the portion of DNA encoding the trans-membrane region in E. coli or 293EBNA cells and purified recombinant sOBM and found that spleen cells differentiated and matured to osteoclasts by the addition of this sOBM in the presence of culture medium of ST2 or M-CSF (Japanese patent applications No. 217897/1997 and No. 224803/1997). From the facts described above, it was found that OCIF and OBM were important factors related specifically to bone metabolism, especially osteoclastogenesis, and in charge of differentiation and maturation of osteoclasts in osteoclastogenesis and that OBM was present in osteoblasts and OCIF suppressed osteoclastogenesis by binding specifically to OBM and blocking the biological activities.

As described above, research and development of a therapeutic agent for metabolic bone diseases, especially osteoporosis, are being vigorously carried out. For studying physiological and pharmacological effects of these agents, it is very important to use animal models of the present invention made on the basis of etiology of osteoporsis. As animal models mainly used for studying effects of agents for osteoporosis at present, ovariectomized female rats which receive artificial operation and exhibits similar pathology to osteoporosis can be exemplified. It is extremely difficult to investigate the effects of an agent for osteoporosis involving various kinds of pathology and factors, using only this ovariectomized rat for analysis. Accordingly, when the effects of an agent for human osteoporosis is investigated in vivo, it is important to use properly several different types of animal models with similar pathology to the disease. Especially, production and usage of pathological model based on the mechanism development of the present disease is important for evaluation of the effects of an agent for the present disease.

Bone remodeling can be divided into 2 phases, bone resorption by osteoclasts and bone formation by osteoblasts. Between these two different kinds of cells, functional coupling is thought to be present and the coupling is thought to be maintained through intercellular response mechanism. Osteoblasts play an important roll in differentiation and maturation of osteoclasts from preosteoclasts by a mechanism involving intercellular adhesion between preosteoclasts and matured osteoclasts (Suda et al.: Bone 17, 87S-91S, 1995; Suda et al.: Endocrine Rev. 13, 66-80, 1992) and in bone resorption by mature osteoclasts (Takahashi et al. Endocrinology Rapid Communication, 2187-2190). Bone formation-stimulating factors (TGF-β, BMP etc.) and growth factors (IGF-I, IGF-II, FGF etc.) were known to be secreted from osteoblasts and thought to play important rolls in maintaining the balance of bone metabolism.

However, many of these factors are multi-functional cytokines which are not specific to bone cells or bone tissues and actions of these cytokines can not be taken as main actions but side-actions. In addition, there is no evidence that the cytokines secreted from osteoblasts are essential factors related to the above-mentioned intercellular responses, that is, differentiation and maturation of osteoclasts and bone resorptive function of matured osteoclasts.

The present inventors already found that fibroblasts and osteoblasts produce and secrete OCIF and that membrane bound protein OBM expressed on osteoblasts is a factor which is related to intercellular signal transduction with preosteoclast and which support and stimulate differentiation and maturation of osteoclasts (Japanese patent applications, No. 217897/1997 and No. 224803/1997). While most of various the cytokines related to bone formation, which were already reported to exhibit multi-functions as described above, OCIF and OBM are cytokines which exhibit only suppressive activity on osteoclastogenesis and stimulative activity on osteoclastogenesis (differentiation and maturation) and they exhibit extremely high specificity to bone cells and bone tissue. Further, while production of OCIF by osteoblasts is down-regulated by active vitamin D₃, parathyroid hormone PTH, prostaglandin E₂, interleukine(IL)-1,6 and 11 which are osteotropic factors essential for osteoclastogenesis, expression of OBM by osteoblasts is up-regulated in the presence of osteotropic factors. It was elucidated that differentiation and maturation of osteoclasts, that is, osteoclastogenesis was regulated through the mechanism described above. From these facts, it was found that OCIF and OBM were essential factors related to osteoclastogenesis in charge of bone resorption.

Human osteoporosis frequently develops in postmenopausal women and is caused by decrease and depletion of female hormone. Decrease and depletion of estrogen induce enhancement of osteoclastsgenesis and break balance between osteoblasts in charge of bone formation and osteoclasts in charge of bone resortpion and bone resorption exceeds over bone formation, resulting in the onset of osteoporosis. Accordingly, transgenic animals deficient of OCIF, in which osteoclastogenesis is enhanced, is expected to become a osteoporotic model which essentially resembles human osteoporosis.

The present inventors eagerly investigated and found that transgenic animals with typical spontaneous osteoporosis and lacking OCIF can be obtained by substituting a portion of an exon encoding the domain essential for expression of OCIF activity in endogenous OCIF gene in the mouse chromosome with the neomycin resistant gene (transgene). That is, an object of the present invention is to provide transgenic animals which can not express endogenous osteoclastogenesis inhibitory factor (OCIF), more specifically which will spontaneously suffer from metabolic bone diseases, especially osteoporosis. Further, another object of the present invention is to provide a method of screening for agents for preventing and/or treating metabolic bone diseases using transgenic animals of the present invention.

### Summary of the Invention

An object of the present invention is to provide transgenic animals which can not express endogenous osteoclastogenesis inhibitory factor (OCIF). Specifically, it is to provide transgenic animals which will spontaneously suffer from metabolic bone diseases, more specifically osteoporosis.

Another object of the present invention is to provide a method of screening agents for preventing and/or treating metabolic bone diseases using transgenic animals of the present invention.

The transgenic animals of the present invention are an ideal model produced by the mechanism involving osteoclastogenesis and the symptom resembles human osteoporosis. Further, because the mortality rate of the animal due to bone fracture of limbs is very low and the survival rate thereof is high, the animal models can grow normally to obtain fecundity and can be bred. Therefore, passage of animal models with spontaneous osteoporosis can be carried out easily by breeding once transgenic animals are made. Since they have advantages as described above, they are useful for research on bone metabolism and/or for evaluating and/or screening agents for preventing and/or treating metabolic bone diseases.

As described above, research and development of therapeutic agents for osteoporosis with higher efficacy and lower side-effects are vigorously being carried out at present. However, animal models mainly used for evaluating effects of agents at present, are female ovariectomized rats received artificial operation and exhibit pathology similar to osteoporosis. It is difficult to investigate the effects of an agent for the present disease using only these animal models. The transgenic animals of the present invention provide a typical osteoporotic model produced by the mechanism of development of the present disease. And they will make it possible to evaluate effects of therapeutic agents for metabolic bone disease and they are also useful for screening for novel therapeutic agents.

### Brief description of the Drawings

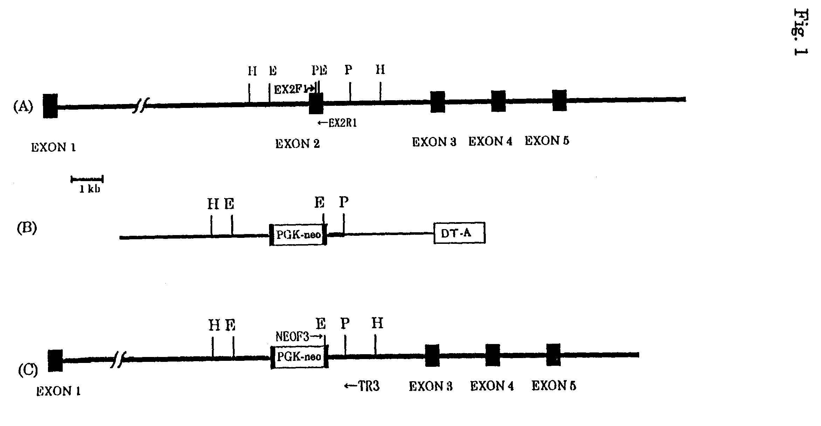

Figure 1 demonstrates homologous recombination in mouse OCIF gene, targeting vector and mouse OCIF gene.

### (A) demonstrates a typical figure of mouse OCIF gene

### Description of codes

■ : exon
― : intron
→ : primer
H : HindIII
E : EcoRI
P : PstI cleavage site

### (B) demonstrates the targeting vector pSKOCIFTarg

### Description of codes

PGK-neo : phosphoglycerate kinase 1 promoter and Neomycin phosphotransferase gene
DT-A : Diphtheria toxin A fragment
H : HindIII
E : EcoRI
P : PstI cleavage site

### (C) demonstrates a typical figure of expected and observed substitution

### Description of codes

PGK-neo : phosphoglycerate kinase 1 promoter and Neomycin phosphotransferase gene
→ : primer
H : HindIII
E : EcoRI
p : PstI cleavage site

Figure 2 demonstrates a photograph of the results of electrophoresis of PCR products in example 7.
A) demonstrates electrophoresis of DNA of 203 bp long
B) demonstrates electrophoresis of DNA of 1157 bp long

### Description of codes

M : molecular weight marker
+/- : heterozygous mouse
+/+ : wild type mouse
-/- : homozygous mouse

Figure 3 demonstrates soft Xray picture of example 8.

Figure 4 demonstrates demineralized specimen of femur epiphysis in example 8.

Figure 5 demonstrates the results of measurement of bone density in example 8.

### Detailed description of the invention and best embodiment for the practice of the present invention

The present invention relates to transgenic animals suffer from spontaneous osteoporosis in which any of the exons (comprising 5 exons, exon 2 to exon 4 encodes domain 1 to domain 4) of OCIF chromosomal gene encoding a domain (from domain 1 to domain 4) essential for expression of OCIF activity is broken and endogenous OCIF can not be produced, and a method of evaluating pharmacological effects of agents for metabolic bone diseases and of screening thereof.

Gene targeting of mouse embryonic stem cell is a powerful method to find out in vivo function of a specific protein. It was clarified that OCIF described in the present invention consisted of 7 domains and domains 1-4 at the N-terminus were essential for the expression of the activity thereof (WO96/26217, WO96/20621).

In addition, the present inventors succeeded in cloning of human and mouse OCIF gene which comprised 5 exons respectively and clarified that exons 2-4 encoded domains 1-4 essential for the expression of activity thereof. The present inventors introduced deletion of transgene, for example neomycin resistant gene, into one of the exon 2 to 4 of mouse OCIF gene using homologous recombination. The exon 2 to 4 of OCIF gene are encoding domains 1-4 essential for the expression of OCIF activity.

In the present invention, it is demonstrated that homozygotic mice do not express OCIF and suffer from typical spontaneous osteoporosis because of this gene conversion. And this transgenic mice are not mortal, grew normally, produced many mice with the same disease and extremely useful as a model of osteoporosis.

A transgenic animals of the present invention can be made by the following method:

### (1) Cloning of OCIF genomic DNA

This is a process of cloning mouse OCIF genomic DNA necessary for preparation of a targeting vector comprising a transgene for homologous recombination. OCIF genomic DNA can be cloned from genomic libraries of E14 ES cells (derived from 129 mouse) using OCIF cDNA as a probe. OCIF genomic DNA obtained like this consists of 5 exons.

### (2) Construction of a targeting vector comprising a transgene necessary for homologous recombination

This is a process of constructing a targeting vector comprising a transgene necessary for homologous recombination. The domains 1-4 which are essential for the expression of biological activity of OCIF are encoded by exons 2-4 of OCIF genomic DNA obtained in process (1). A transgene which can not encode OCIF can be prepared by deleting any part of these exons or by inserting foreign gene, and a targeting vector comprising this transgene can be constructed. Though vectors used here are not especially restricted, for example, pBR 322 which can work in E. coli., pUB110 which can work in Bacillus subtitles or pSG5 and pXT1 which can work in animal cells may be used.

### (3) Introduction of the targeting vector into ES cells

The targeting vector obtained in process (2) is introduced into E14 ES cells by electroporation method.

### (4) Selection of ES cells wherein the transgene was integrated into the chromosome DNA

ES cells in which a part of OCIF genomic DNA was substituted with the transgene prepared in process (2) by homologous recombination can be selected by polymerase chain reaction (PCR) method and it can be confirmed by Southern blotting that a part of OCIF gene is substituted with the transgene as expected and there is no illegitimate recombination into other sites.

### (5) Transplantation of ES cells in which OCIF gene was substituted with the transgene into uterus of pseudopregnant females and preparation of transgenic animals

This is a process of injecting ES cell selected in process (4) into embryos at the blastocyst stage, transplanting the embryos into uterus of pseudopregnant females and prepare transgenic animals. Transgenic animals can be confirmed by analyzing contribution rate of ES cell in body hair and by Southern blotting. Homozygous transgenic animals can be prepared by mating heterozygous transgenic animals.

The transgenic animals of the present invention obtained in this manner lacks OCIF by knockout of the gene of osteoclastogenesis inhibitory factor (OCIF) and will suffer from spontaneous osteoporosis. Especially, the animals of the present invention is model animals of osteoporosis, having the following excellent characters: 1) There are ideal animal model produced based on the mechanism of bone metabolism, especially, osteoclastogenesis. There are model animals with spontaneous osteoporosis induced by enhanced osteoclastogenesis and excessive bone resorption in which the gene of osteoclastogenesis inhibitory factor, OCIF (WO96/26217, WO96/20621), which is essential in bone metabolism, is knocked out. Further, it is known that enhanced osteoclastogenesis and exceeding of bone resoption over bone formation will induce osteoporosis in postmenopausal women and the mechanism of onset of osteoporosis in the animals of the present invention is quite similar to that of humans as described above. 2) The mortality rate of the model animals with spontaneous osteoporosis of the present invention due to severe symptoms (bone fracture of limb etc.) is about 10% of all the younglings. Most of the remaining animals exhibit osteoporosis loss of body weight in the transgenic animals is insignificant as compared to normal animals and can be used as animal modelss. Further, 3) More than 50 % of neonates can grow and breed. Therefore, once transgenic animals are prepared, desired number of animal modelss with spontaneous osteoporosis can be bred.

As described above, the transgenic animals of the present invention have excellent characteristics as model animals with spontaneous osteoporosis. Accordingly, the transgenic animals with spontaneous osteoorosis of the present invention can be used for evaluation of agents for human osteoporosis which are being developed as therapeutic agents for metabolic bone diseases, mostly ostoporosis. That is, the effects of these agents can be evaluated by administrating thereof into the transgenic animals of the present invention and investigating improvement of bone density and bone strength. In addition, the transgenic animals of the present invention is useful for in vivo screening of novel therapeutic agents for metabolic bone diseases.

### [Example]

The present invention will be described more specifically by exhibiting the following examples but these are only exemplification and the present invention will not be restricted by these examples.

### [Example 1]

### Cloning of mouse OCIF genomic DNA

Genomic library was prepared by extracting genomic DNA of mouse ES cell line E14, digesting partially the genomic DNA with restriction enzyme Sau3AI and inserting cleaved DNA into phage vector λ DASH II (Stratagene). From this library, 5 phage clones were isolated by plaque hybridization method using OCIF cDNA as a probe. Inserts of these 5 phage clones was digested by restriction enzymes described in figure 1 and analyzed by Southern blotting, and then subcloned at multi-cloning sites of plasmid vector pBluescriptSK(+) (hereinafter abbreviated as pSK, Stratagene). The structure of mouse OCIF genomic DNA was clarified by Southern blotting analysis and analysis of base sequence. As figure 1 shows, mouse OCIF genomic DNA spans about 30 kb and has 5 exons. The exon 2-4 encode domains 1-4 essential for the expression of OCIF activity.

### [Example 2]

### Transgene and construction of a targeting vector

A targeting vector was constructed for disrupting exon 2 of mouse OCIF genomic DNA. That is, using subclones prepared in example 1, pSK2H4-2 (4.5 kb of DNA fragment comprising exon 2) and pSK2E5 (about 5 kb of DNA fragment from 3' of intron 1), about 6 kb DNA fragment in upper stream from PstI site of exon 2 and about 1 kb of DNA fragment in down stream from the PstI site were isolated. Further, 1.3 kb of neo cassette DNA fragment (neo resistance gene in down stream from PGK promoter) was also isolated from pKJ2 (Popo H. et al., Biochemical Genetics, 28, 299-308 (1990), Lifetech Oirental). Then vector pSKT (-DT) was prepared by connecting the above 3 DNA fragments in order of about 6 kb DNA fragment, neo cassette DNA fragment and the 1 kb DNA fragment and inserting this connected DNA (about 8.3 kb) into pSK which was cleaved with SacI and BamHI (Takara) in advance.

From this pSKT (-DT), 7.3 kb of SalI-BamHI DNA fragment and 1 kb of BamHI-HindIII DNA fragment were isolated. A targeting vector pSKOCIFTarg as the final product was prepared by cleaving pMCDT-A (A+T/pau) (Lifetech Oriental) with SalI and HindIII and ligating the three with DNA ligase (Takara). The pSKOCIFTarg has neo resistance gene as positive selection marker in exon 2 of OCIF gene and diphtheria toxin A fragment gene (DT-A) as negative selection marker outside of short arm of OCIF homologous region as shown in figure 1(B). pSKTOCIFTarg can become linear with SalI. E. coli. transformed with the targeting vector of figure 1(B) of the present invention was deposited with deposit number of FERM BP-6153 at National Institute of Bioscience and Human-Technology Agency of Industrial Science and Technology Ministry of International Trade and Industry on October 23, 1997.

### [Example 3]

### Culture of ES cell and introduction of the targeting vector into ES cell

A cell line for feeder layer derived from mouse fibroblast was cultured using DMEM (Dulbecco's Modified Eagle Medium, GIBCO BRL) 10% bovine fetal serum at 37 °C under 5% CO₂. Then ES cell line E14 was cultured on the feeder layer pretreated with Mitomycin C using a medium for ES cell which was modified DMEM plus 20 % fetal calf serum (Robertson E. J.: Teratocarcinomas and Embryonic Stem Cells, pp. 71-112, IRL press, 1987) at 37 °C under 5 % CO₂. ES cells under growth was deprived from culture dish by trypsin treatment and made a single cell suspension. After centrifugation, the cells were suspended in phosphate buffer solution at the concentration of 1.25 x 10⁷/ml. A targeting vector was introduced into ES cells by mixing 0.8ml ES cells suspension with 25 µg of targeting vector linearized with SalI in example 2 and placing it into 0.4 cm cuvette, followed by electroporation (220-250 v, 500 F). Gene pulser of Bio-Rad was used for electroporation. After then, cells from each cuvette were dispersed homogeneously on a dish with 3 feeder layers (6cm), and cultured in a medium for ES cells. Next day, after the culture medium was exchanged with the same fresh medium, G418 (GIBCO BRL) was added so as to be 250 µg/ml. It was cultured by exchanging the medium containing the same concentration of G418 daily until 7-10 days after electroporation. ES cell colonies as resistant, clones were picked up 7-10 days after, treated with trypsin, and made a suspension. A half of the suspension was transferred to a 48 well plate with feeder layer, the remaining half thereof was placed into a centrifugation tube. Cell lysate of them was prepared for a sample of polymerize chain reaction (PCR). The ES cells on 48 well plate was cultured in a medium for ES cells.

### [Example 4]

### DNA analysis of ES cell integrated with the targeting vector.

Identification of ES cell clone after homologous recombination was carried out by PCR. Centrifugation tubes comprising ES cells collected in example 3 were centrifugated (1000 rpm x 10 min.), removed the supernatant and mixed with 40 µl of sterilized distilled water. Mineral oil (Sigma) was put into the cell suspension in centrifugation tubes, which were kept at 95 °C for 10 minutes then at 55 °C. Thereto, 10 µl of 1 mg/ml proteinase K(MERCK) was added and treated with enzyme at 55 °C for 60-120 minutes, and then it was inactivated at 95 °C for 10 minutes and kept at 4 °C. Cell suspension after the addition of 2 steps distilled water or cell lysate after enzymatic treatment was kept at lower than -20 °C when the next procedure was not taken. One pair of primer set, that is, NEOF3 (a sense primer specific to neo gene close to 3' terminus of neo gene: Sequence identification number 1) and TR3 (an antisense primer specific to mouse OCIF gene present on the genomic DNA in down stream from 3' terminus of targeting vector; sequence identification number 2) which can amplify 1157 bp of DNA fragment specific to mouse OCIF gene after the objective homologous recombination were prepared by a DNA synthesizer. PCR was carried out using recombinant Taq polymerase (Takara) and attached buffer solution in total 40 µl containing 20 µl of cell lysate treated with enzyme as above, 4 µl of the same buffer of 10 times concentration without Mg, 3.2 µl of 10 mM dTNP, 4 µl of 25 mM MgCl₂, 2 µl of DMSO, 1 µl of 20 µM each primer, 0.4 µl of Taq polymerase and 4.4 µl of 2 steps distilled water. The reaction was repeated 35 times , wherein one cycle consisted of 95 °C for 30 seconds, 58 °C for 1 minute and 72 °C for 1 minute. PCR products were analyzed by electrophoresis using agarose gel and the presence or absence of specific band was confirmed. Only ES clones which were judged as positive were cultured for growth and stored in liquid nitrogen. DNA was extracted from the cells using DNAzol (GIBCO BRL) according to the attached explanation, digested with restriction enzyme and examined for confirmation of homologous recombination and of the absence of illegitimate recombination by Southern blotting. By this series of methods, targeted ES clone has homologous recombinant OCIF gene as shown in figure 1 (C).

### [Example 5]

### Introduction of ES cells into embryos and embryo transfer

To induce superovulation, 5 IU of each of pregnant mare's serum gonadotropin (PMSG) and human chorionic gondotropin (hCG) were intraperitoneally administered at interval of 48 hours in female C57BL/6N or B6C3F1 (F1 of C57BL/6N with C3H/He) mice. The female mice were mated with the same line of male mice after the hcg administration. Then fertilized eggs was collected from oviducts or uterine horns of the female mice by flushing with M2 medium (Sigma). The all fertilized ovum collected at any time were cultured up to blastocyst period in M16 medium at 37 °C under 5 % CO₂.

Pre-cultured targeted ES cells with homologous recombination were treated with trypsin to make single cell suspension and 10-15 ES cells per blastocyst were injected into blastocoelic cavity by micromanipulation. Youngborn was obtained by transplanting the blastocysts injected with ES cells into uterine horn of pseudopregnant female ICR mice.

### [Example 6]

### Selection of heterozygous mice for mutated OCIF allele

Chimeric mice with somatic cell derived from ES cell were selected among the youngborn. Since ES cell line E14 was derived from 129/01a mice having light yellow body hair and red eyes, chimeric mice can be easily detected by looking at the coat color and eyes. Male chimeric mice were mated with female C57BL/6N mice or female ICR mice and OCIF-mutated heterozygous(+/-) with homologous recombinant gene were screened by PCR method. Templates used in PCR were prepared according to a method of Chen S and Evans G.A. (Method in Molecular Biology, 15, 75-80 (1993)). That is, holes for discrimination of individual were made in ears of mice using an earpunch for small animal and tissue pieces with diameter of 1-2 mm obtained at the same time were collected. The specimens were treated with proteinase K(MERCK) and was diluted with distilled water to make templates of PCR. PCR was carried out using primer NEOF3 (Sequence identification number 1) and TR3 (sequence identification number 2) according to a method described in example 4 except that the amount of templates added to the reaction mixture was less than one twentieth of the liquid volume. As to whether or not it was a heterozygous mouse was determined by electrophoresis of the PCR products. As the results of this determination, chimeric mice derived from 2 independent ES cell lines were transmitting the mutated genotype to their off springs. Apparent specified abnormality was not observed in both lines of mice.

### [Example 7]

### Production of homozygous of transgenic mice and DNA analysis

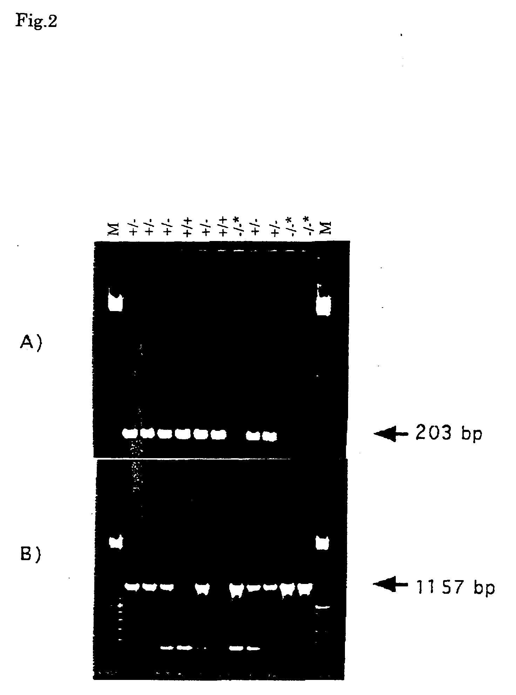

Next generation mice were obtained by mating the same line of OCIF mutated heterozygous mice. Analysis of genotype of next generation mice was carried out according to partially improved method described in example 6. In order to examine the presence of normal OCIF gene, PCR was carried out using primer EX2F1 (Sequence identification number 3) and primer EX2R1 (Sequence identification number 4) which could amplify 203 bp of DNA fragment specific to exon 2 of mouse OCIF gene. The results were shown in figure 2. As a result, 203 bp of clear band was observed from PCR of wild type(+/+) having only normal OCIF gene using primer set of EX2F1-EX2R1. In the cases of heterozygous (+/-) which were recombinated and had one normal OCIF, 1157 bp and 203 bp of clear bands were observed from NEOF3-TR3 and EX2F1-EX2R1, respectively.

And in the case of homozygous (that is, knockout mouse) (-/-) having not normal OCIF gene, the band was observed only with NEOF3-TR3. In addition, genotype of these OCIF was confirmed by Southern blot, analysis. As the results of genotype analysis, it was clarified that OCIF mutation comprised genotypes of hetero (+/-), wild (+/+) and homo (-/-). The inactivation of OCIF gene did not induce lethality during development. After birth, about 10-20 % of OCIF mutated homo-mice died due to debility before weaning and abnormal bending of limbs was clearly observed by clinical observation. About 80 % of OCIF mutated homo-mice was confirmed to grow up without specific anomaly. As the results of northern blotting, OCIF mRNA was not expressed in homo-mice.

### [Example 8]

### Phenotype of OCIF knockout mice

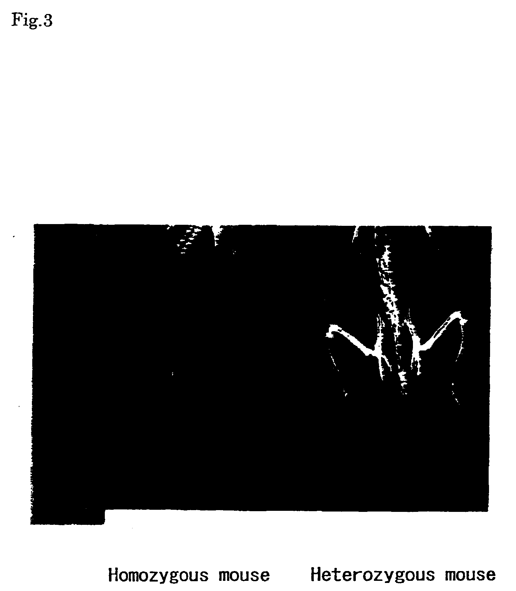

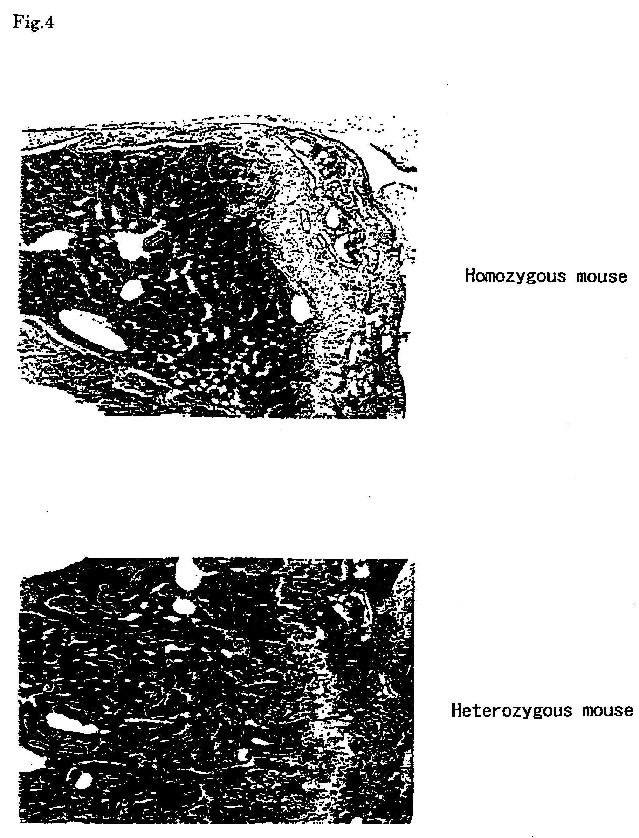

OCIF knockout mice were analyzed anatomically and histopathologically. Mice were anesthetized deeply with diethylether and soft Xray radiograph thereof was taken, followed by exanguination to death and autopsy thereof. Femur and tibia were excised, fixed with 4 % paraformaldehyde, 1 % glutalaldehyde, decalcified and stained with hematoxyl in eosin to make specimen. Another femur and lumber vertebrae were fixed in alcohol and skin and muscle were removed, followed by measure ment of bone density. Analysis by soft Xray radiography was shown in figure 3. As the results, the density of Xray radiograph in systemic bone tissue was quite low in homomice. Homozygous (-/-) showed weaker image of femur, tibia and caudal vertebrae than that of heterozygous (+/-). Difference was distinguished in femur epiphysis and lumber vertebrae. In addition, as shown in figure 4, trabecular bone, that is, cancerous bone was present in internal capital of femur in wild mice (+/+), while that was scarcely present and bone marrow occupied femur interior in homozygous (-/-). Further, bone mineral density (hereinafter referred to BMD) of femur epiphysis in OCIF homozygous (-/-), heteromice (+/-) and wild-type mice (+/+) were determined.

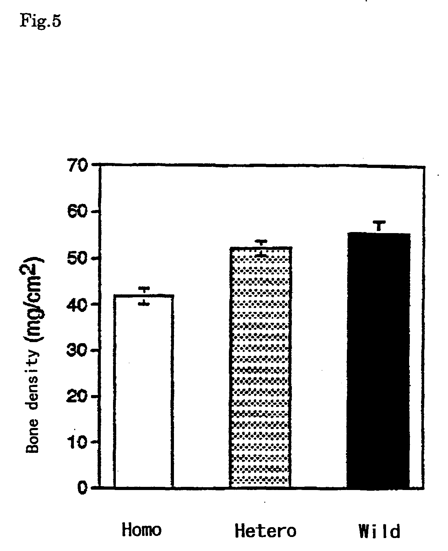

The results were shown in figure 5. As the results, BMD in OCIF gene deleted homomice was significantly lower than those of heteromice and wild mice. From these observations, OCIF knockout mice (-/-) was found to suffer from spontaneous osteoporosis. However, no anomaly was observed in the other tissues of OCIF knockout mice.

### [Example 9]

### Fertility of OCIF knockout mice

Fertility of OCIF knockout mice (-/-) was confirmed by mating experiments. Among mice with genetic background of ICR mice, 8-12 weeks old male homozygous (-/-) without apparent anomaly were mated with the same line of female heterozygous (+/-) or female homozygous (-/-). As the results, youngborn were obtained from the all pairs. Total of 38 neonates were born from 4 pairs of homozygous and 24 (63 %) of them survived after 4 weeks. On the other hand, 59 neonates were bone from 6 pairs with female heterozygous and 51 (90 %) of them survived after 4 weeks. From the results, though the survival rate of neonates in the case of production and raising by female homozygous was lower than that of female heterozygous, it was confirmed that about 60 % of neonates from female homozygous survived after weaning. Further, sex ratio of grown up homozygous was close to 1:1 and difference of survival rate coming from sex difference was not observed.

## Claims

1. A transgenic animal which can not express endogenous osteoclastogenesis inhibitory factor (OCIF).

2. The transgenic animal according to claim 1 which will suffer from metabolic bone disease.

3. The transgenic animal according to claim 2 wherein said metabolic bone disease is osteoporosis.

4. The transgenic animal according to claim 1-3 wherein said animal is mouse.

5. A method of screening an agent for preventing and/or treating metabolic bone disease characterized by using the transgenic animal described in claim 1-4.


Sequence Listing

<110> Snow Brand Milk Products Co., Ltd.

<120> Transgenic animal

<130> YTP97032

<160> 4

<170> Microsoft Word

<210> 1
<211> 25
<212> DNA

<223> Synthetic DNA

<400> 1
atcgccttct atcgccttct tgacg

<210> 2
<211> 25
<212> DNA

<223> Synthetic DNA

<400> 2
gagcaaatgg acctcccgta atgga

<210> 3
<211> 25

<212> DNA

<223> Synthetic DNA

<400> 3
tcctggcacc tacctaaaac agcac

<210> 4
<211> 24
<212> DNA

<223> Synthetic DNA

<400> 4
ggtaacgccc ttcctcacac tcac